# EUROPEAN PATENT SPECIFICATION

(11) **EP 0 858 509 B1**
(45) Date of publication and mention of the grant of the patent: **27.02.2002**
(21) Application number: 96937095.6
(22) Date of filing: 01.11.1996
(51) Int. Cl.: C12N 15/52, C12N 9/64, C07K 16/40

(54) **DNA SEQUENCES ENCODING CMH-1, A CYSTEINE PROTEASE INVOLVED IN APOPTOSIS**
IN APOPTOSIS BETEILIGTE CYSTEINE PROTEASE CMH-1 UND DNS-SEQUENZEN DAFÜR
SEQUENCES D'ADN CODANT POUR LA CMH-1, UNE CYSTEINE PROTEASE IMPLIQUEE DANS L'APOPTOSE

(30) Priority: 03.11.1995 US 7211 P; 06.11.1995 US 7251 P; 16.11.1995 US 558733
(43) Date of publication of application: 19.08.1998
(73) Proprietor: VERTEX PHARMACEUTICALS INCORPORATED, Cambridge, MA 02139-4242 (US)
(72) Inventor: SU, Michael, Newton, MA 02159 (US); LIPPKE, Judith, A., Roslindale, MA 02131 (US)
(74) Representative: Schlich, George William
(86) International application number: US9617431
(87) International publication number: WO9716552

(56) References cited:
- WO-A-96/13603
- J. BIOL CHEM. , vol. 269, no. 49, 9 December 1994, pages 30761-30764, XP000616157 T. FERNANDES-ALNEMRI ET AL.: "CPP32, a novel human apoptotic protein with homology to Caenorhabditis elegans cell death protein Ced-3 and mammalian Interleukin-1beta-converting enzyme." cited in the application
- CELL, vol. 81, 2 June 1995, pages 801-809, XP002013933 M. TEWARI ET AL.: "Yama/CPP32beta, a mammalian homolog of CED-3, is a CrmA-inhibitable protease that cleaves the death substrate poly{ADP-ribose} polymerase." cited in the application
- NATURE, vol. 376, 6 July 1995, pages 37-43, XP000574812 D.W. NICHOLSON ET AL.: "Identification and inhibition of the ICE/CED-3 protease necessary for mammalian apoptosis." cited in the application
- JOURNAL OF BIOLOGICAL CHEMISTRY 271 (4). 1996. 1825-1828. ISSN: 0021-9258, 26 January 1996, XP000616154 LIPPKE J A ET AL: "Identification and characterization of CPP32- Mch2 homolog 1, a novel cysteine protease similar to CPP32."
- CANCER RESEARCH 55 (24). 1995. 6045-6052. ISSN: 0008-5472, 15 December 1995, XP000614740 FERNANDES-ALNEMRI T ET AL: "Mch3, a novel human apoptotic cysteine protease highly related to CPP32."
- J. BIOL. CHEM. (1996), 271(3), 1621-5 CODEN: JBCHA3;ISSN: 0021-9258, 19 January 1996, XP000616150 DUAN, HANGJUN ET AL: "ICE - LAP3, a novel mammalian homolog of the Caenorhabditis elegans cell death protein Ced-3 is activated during Fas- and tumor necrosis factor-induced apoptosis"
- PROCEEDINGS OF THE NATIONAL ACADEMY OF SCIENCES OF THE UNITED STATES OF AMERICA 93 (11). 1996. 5437-5442. ISSN: 0027-8424, 28 May 1996, XP000616168 PAI J-T ET AL: "Purification and cDNA cloning of a second apoptosis-related cysteine protease that cleaves and activates sterol regulatory element binding proteins."
- JOURNAL OF BIOLOGICAL CHEMISTRY 271 (18). 1996. 10816-10820. ISSN: 0021-9258, 3 May 1996, XP000616152 GU Y ET AL: "Processing and activation of CMH -1 by granzyme B."

### SEQUENCE LISTING

(1) GENERAL INFORMATION:
   (i) APPLICANT:
      (A) NAME: Vertex Pharmaceuticals Incorporated
      (B) STREET: 130 Waverly Street
      (C) CITY: Cambridge
      (D) STATE: Massachusetts
      (E) COUNTRY: United States of America
      (F) POSTAL CODE (ZIP): 02139-4242
      (G) TELEPHONE: 617-577-6000
      (H) TELEFAX: 617-577-6680
   (ii) TITLE OF INVENTION: DNA SEQUENCES ENCODING CMH-1, A CYSTEINE PROTEASE INVOLVED IN APOPTOSIS
   (iii) NUMBER OF SEQUENCES: 11
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: US 60/007,211
      (B) FILING DATE: 03-NOV-1995
   (vi) PRIOR APPLICATION DATA:
      (A) APPLICATION NUMBER: US 60/007,251
      (B) FILING DATE: 06-NOV-1995
   (vi) PRIOR APPLICATION DATA:
      (A) APPLICATION NUMBER: US 08/558,733
      (B) FILING DATE: 16-NOV-1995
(2) INFORMATION FOR SEQ ID NO:1:
   (i) SEQUENCE CHARACTERISTICS:
      (A) LENGTH: 2341 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 54..962
      (D) OTHER INFORMATION: /function= "cysteine protease" /product= "CMH-1"
   (xi) SEQUENCE DESCRIPTION: SEQ ID NO:1:
   CTAATGCGGA GAGAGACTGT GCCAGTCCCA GCCGCCCTAC CGCCGTGGGA ACG ATG      56
                                                             Met
                                                              1
   GCA GAT GAT CAG GGC TGT ATT GAG GAG CAG GGG GTT GAG GAT TCA GCA    104
   Ala Asp Asp Gln Gly Cys Ile Glu Glu Gln Gly Val Glu Asp Ser Ala
               5                  10                  15
   AAT GAA GAT TCA GTG GAT GCT AAG CCA GAC CGG TCC TCG TTT GTA CCG    152
   Asn Glu Asp Ser Val Asp Ala Lys Pro Asp Arg Ser Ser Phe Val Pro
           20                  25                  30
   TCC CTC TTC AGT AAG AAG AAG AAA AAT GTC ACC ATG CGA TCC ATC AAG    200
   Ser Leu Phe Ser Lys Lys Lys Lys Asn Val Thr Met Arg Ser Ile Lys
       35                  40                  45
   ACC ACC CGG GAC CGA GTG CCT ACA TAT CAG TAC AAC ATG AAT TTT GAA    248
   Thr Thr Arg Asp Arg Val Pro Thr Tyr Gln Tyr Asn Met Asn Phe Glu
    50                  55                  60                  65
   AAG CTG GGC AAA TGC ATC ATA ATA AAC AAC AAG AAC TTT GAT AAA GTG    296
   Lys Leu Gly Lys Cys Ile Ile Ile Asn Asn Lys Asn Phe Asp Lys Val
                   70                  75                  80
   ACA GGT ATG GGC GTT CGA AAC GGA ACA GAC AAA GAT GCC GAG GCG CTC    344
   Thr Gly Met Gly Val Arg Asn Gly Thr Asp Lys Asp Ala Glu Ala Leu
               85                  90                  95
   TTC AAG TGC TTC CGA AGC CTG GGT TTT GAC GTG ATT GTC TAT AAT GAC    392
   Phe Lys Cys Phe Arg Ser Leu Gly Phe Asp Val Ile Val Tyr Asn Asp
           100                 105                 110
   TGC TCT TGT GCC AAG ATG CAA GAT CTG CTT AAA AAA GCT TCT GAA GAG    440
   Cys Ser Cys Ala Lys Met Gln Asp Leu Leu Lys Lys Ala Ser Glu Glu
       115                 120                 125
   GAC CAT ACA AAT GCC GCC TGC TTC GCC TGC ATC CTC TTA AGC CAT GGA    488
   Asp His Thr Asn Ala Ala Cys Phe Ala Cys Ile Leu Leu Ser His Gly
   130                 135                 140                 145
   GAA GAA AAT GTA ATT TAT GGG AAA GAT GGT GTC ACA CCA ATA AAG GAT    536
   Glu Glu Asn Val Ile Tyr Gly Lys Asp Gly Val Thr Pro Ile Lys Asp
                   150                 155                 160
   TTG ACA GCC CAC TTT AGG GGG GAT AGA TGC AAA ACC CTT TTA GAG AAA    584
   Leu Thr Ala His Phe Arg Gly Asp Arg Cys Lys Thr Leu Leu Glu Lys
               165                 170                 175
   CCC AAA CTC TTC TTC ATT CAG GCT TGC CGA GGG ACC GAG CTT GAT GAT    632
   Pro Lys Leu Phe Phe Ile Gln Ala Cys Arg Gly Thr Glu Leu Asp Asp
           180                 185                 190
   GGC ATC CAG GCC GAC TCG GGG CCC ATC AAT GAC ACA GAT GCT AAT CCT    680
   Gly Ile Gln Ala Asp Ser Gly Pro Ile Asn Asp Thr Asp Ala Asn Pro
       195                 200                 205
   CGA TAC AAG ATC CCA GTG GAA GCT GAC TTC CTC TTC GCC TAT TCC ACG    728
   Arg Tyr Lys Ile Pro Val Glu Ala Asp Phe Leu Phe Ala Tyr Ser Thr
   210                 215                 220                 225
   GTT CCA GGC TAT TAC TCG TGG AGG AGC CCA GGA AGA GGC TCC TGG TTT    776
   Val Pro Gly Tyr Tyr Ser Trp Arg Ser Pro Gly Arg Gly Ser Trp Phe
                       230                      235                      240
   GTG CAA GCC CTC TGC TCC ATC CTG GAG GAG CAC GGA AAA GAC CTG GAA      824
   Val Gln Ala Leu Cys Ser Ile Leu Glu Glu His Gly Lys Asp Leu Glu
               245                      250                      255
   ATC ATG CAG ATC CTC ACC AGG GTG AAT GAC AGA GTT GCC AGG CAC TTT      872
   Ile Met Gln Ile Leu Thr Arg Val Asn Asp Arg Val Ala Arg His Phe
           260                      265                      270
   GAG TCT CAG TCT GAT GAC CCA CAC TTC CAT GAG AAG AAG CAG ATC CCC      920
   Glu Ser Gln Ser Asp Asp Pro His Phe His Glu Lys Lys Gln Ile Pro
       275                      280                      285
   TGT GTG GTC TCC ATG CTC ACC AAG GAA CTC TAC TTC AGT CAA              962
   Cys Val Val Ser Met Leu Thr Lys Glu Leu Tyr Phe Ser Gln
   290                      295                      300
   TAGCCATATC AGGGGTACAT TCTAGCTGAG AAGCAATGGG TCACTCATTA ATGAATCACA 1022
   TTTTTTTATG CTCTTGAAAT ATTCAGAAAT TCTCCAGGAT TTTAATTTCA GGAAAATGTA 1082
   TTGATTCAAC AGGGAAGAAA CTTTCTGGTG CTGTCTTTTG TTCTCTGAAT TTTCAGAGAC 1142
   TTTTTTTATA ATGTTATTCA TTTGGTGACT GTGTAACTTT CTCTTAAGAT TAATTTTCTC 1202
   TTTGTATGTC TGTTACCTTG TTAATAGACT TAATACATGC AACAGAAGTG ACTTCTGGAG 1262
   AAAGCTCATG GCTGTGTCCA CTGCAATTGG TGGTAACAGT GGTAGAGTCA TGTTTGCACT 1322
   TGGCAAAAAG AATCCCAATG TTTGACAAAA CACAGCCAAG GGGATATTTA CTGCTCTTTA 1382
   TTGCAGAATG TGGGTATTGA GTGTGATTTG AATGATTTTT CATTGGCTTA GGGCAGATTT 1442
   TCATGCAAAA GTTCTCATAT GAGTTAGAGG AGAAAAAGCT TAATGATTCT GATATGTATC 1502
   CATCAGGATC CAGTCTGGAA AACAGAAACC ATTCTAGGTG TTTCAACAGA GGGAGTTTAA 1562
   TACAGGAAAT TGACTTACAT AGATGATAAA AGAGAAGCCA AACAGCAAGA AGCTGTTACC 1622
   ACACCCAGGG CTATGAGGAT AATGGGAAGA GGTTTGGTTT CCTGTGTCCA GTAGTGGGAT 1682
   CATCCAGAGG AGCTGGAACC ATGGTGGGGG CTGCCTAGTG GGAGTTAGGA CCACCAATGG 1742
   ATTGTGGAAA ATGGAGCCAT GACAAGAACA AAACCACTGA CTGAGATGGA GTGAGCTGAG 1802
   ACAGATAAGA GAATACCTTG GTCTCACCTA TCCTGCCCTC ACATCTTCCA CCAGCACCTT 1862
   ACTGCCCAGG CCTATCTGGA AGCCACCTCA CCAAGGACCT TGGAAGAGCA AGGGACAGTG 1922
   AGGCAGGAGA AGAACAAGAA ATGGATGTAA GCCTGGCCCA TAATGTGAAC ATAAGTAATC 1982
   ACTAATGCTC AACAATTTAT CCATTCAATC ATTTATTCAT TGGGTTGTCA GATAGTCTAT 2042
   GTATGTGTAA AACAATCTGT TTTGGCTTTA TGTGCAAAAT CTGTTATAGC TTTAAAATAT 2102
   ATCTGGAACT TTTTAGATTA TTCCAAGCCT TATTTTGAGT AAATATTTGT TACTTTTAGT 2162
   TCTATAAGTG AGGAAGAGTT TATGGCAAAG ATTTTTGGGC ACTTTGTTTT CAAGATGGTG 2222
   TTATCTTTTG AATTCTTGAT AAATGACTGT TTTTTTCTGC CTAATAGTAA CTGGTTAAAA 2282
   AACAAATGTT CATATTTATT GATTAAAAAT GTGGTTGCTT AAAAAAAAAA AAAAAAAAA 2341
(2) INFORMATION FOR SEQ ID NO:2:
   (i) SEQUENCE CHARACTERISTICS:
      (A) LENGTH: 303 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Ala Asp Asp Gln Gly Cys Ile Glu Glu Gln Gly Val Glu Asp Ser
   1 5 10 15
   Ala Asn Glu Asp Ser Val Asp Ala Lys Pro Asp Arg Ser Ser Phe Val
   20 25 30
   Pro Ser Leu Phe Ser Lys Lys Lys Lys Asn Val Thr Met Arg Ser Ile
   35 40 45
   Lys Thr Thr Arg Asp Arg Val Pro Thr Tyr Gln Tyr Asn Met Asn Phe
   50 55 60
   Glu Lys Leu Gly Lys Cys Ile Ile Ile Asn Asn Lys Asn Phe Asp Lys
   65 70 75 80
   Val Thr Gly Met Gly Val Arg Asn Gly Thr Asp Lys Asp Ala Glu Ala
   85 90 95
   Leu Phe Lys Cys Phe Arg Ser Leu Gly Phe Asp Val Ile Val Tyr Asn
   100 105 110
   Asp Cys Ser Cys Ala Lys Met Gln Asp Leu Leu Lys Lys Ala Ser Glu
   115 120 125
   Glu Asp His Thr Asn Ala Ala Cys Phe Ala Cys Ile Leu Leu Ser His
   130 135 140
   Gly Glu Glu Asn Val Ile Tyr Gly Lys Asp Gly Val Thr Pro Ile Lys
   145 150 155 160
   Asp Leu Thr Ala His Phe Arg Gly Asp Arg Cys Lys Thr Leu Leu Glu
   165 170 175
   Lys Pro Lys Leu Phe Phe Ile Gln Ala Cys Arg Gly Thr Glu Leu Asp
   180 185 190
   Asp Gly Ile Gln Ala Asp Ser Gly Pro Ile Asn Asp Thr Asp Ala Asn
   195 200 205
   Pro Arg Tyr Lys Ile Pro Val Glu Ala Asp Phe Leu Phe Ala Tyr Ser
   210 215 220
   Thr Val Pro Gly Tyr Tyr Ser Trp Arg Ser Pro Gly Arg Gly Ser Trp
   225 230 235 240
   Phe Val Gln Ala Leu Cys Ser Ile Leu Glu Glu His Gly Lys Asp Leu
   245 250 255
   Glu Ile Met Gln Ile Leu Thr Arg Val Asn Asp Arg Val Ala Arg His
   260 265 270
   Phe Glu Ser Gln Ser Asp Asp Pro His Phe His Glu Lys Lys Gln Ile
   275 280 285
   Pro Cys Val Val Ser Met Leu Thr Lys Glu Leu Tyr Phe Ser Gln
   290 295 300
(2) INFORMATION FOR SEQ ID NO:3:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR oligonucleotide primer"
   (iii) HYPOTHETICAL: NO
   (iv) ANTI-SENSE: NO
   (xi) SEQUENCE DESCRIPTION: SEQ ID NO:3:
   CCATCCTAAT ACGACTCACT ATAGGGC                                27
(2) INFORMATION FOR SEQ ID NO:4:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer oligonucleotide"
   (iii) HYPOTHETICAL: NO
   (iv) ANTI-SENSE: NO
   (xi) SEQUENCE DESCRIPTION: SEQ ID NO:4:
   GCAAACTCTG TCAATTCACC C                                      21
(2) INFORMATION FOR SEQ ID NO:5:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR oligonucleotide primer"
   (iii) HYPOTHETICAL: NO
   (iv) ANTI-SENSE: NO
   (xi) SEQUENCE DESCRIPTION: SEQ ID NO:5:
   GCTGCAGTCT AGAGCTCCAT GGCTAGCATG ACTGGTGGAC AGCAAATGGG TGCTAAGCCA 60
   GACCGGTCCT CG 72
(2) INFORMATION FOR SEQ ID NO:6:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR oligonucleotide primer"
   (iii) HYPOTHETICAL: NO
   (iv) ANTI-SENSE: NO
   (xi) SEQUENCE DESCRIPTION: SEQ ID NO:6:
   GAATTCTAGA TTCTATTGAC TGAAGTAGAG TTC 33
(2) INFORMATION FOR SEQ ID NO:7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "site-directed mutagenesis oligonucleotide"
   (iii) HYPOTHETICAL: NO
   (iv) ANTI-SENSE: NO
   (xi) SEQUENCE DESCRIPTION: SEQ ID NO:7:
   CATTCAGGCT AGCCGAGGGA C 21
(2) INFORMATION FOR SEQ ID NO:8:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "site-directed mutagenesis oligonucleotide"
   (iii) HYPOTHETICAL: NO
   (iv) ANTI-SENSE: NO
   (xi) SEQUENCE DESCRIPTION: SEQ ID NO:8:
   GCTATTACTC AGCTGAGAGC CCAGGA 26
(2) INFORMATION FOR SEQ ID NO:9:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer oligonucleotide"
   (iii) HYPOTHETICAL: NO
   (iv) ANTI-SENSE: NO
   (xi) SEQUENCE DESCRIPTION: SEQ ID NO:9:
   CGGCTCGAGG CTAAGCCAGA CCGGTCCTCG 30
(2) INFORMATION FOR SEQ ID NO:10:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR primer oligonucleotide"
   (iii) HYPOTHETICAL: NO
   (iv) ANTI-SENSE: NO
   (xi) SEQUENCE DESCRIPTION: SEQ ID NO:10:
   GGGCTCGAGC TATTGACTGA AGTAGAGTTC 30
(2) INFORMATION FOR SEQ ID NO:11:
   (i) SEQUENCE CHARACTERISTICS:
      (A) LENGTH: 23 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (xi) SEQUENCE DESCRIPTION: SEQ ID NO:11:
   Met Gly Ser Ser His His His His His His Ser Ser Gly Leu Val Pro
   1               5                   10                  15
   Arg Gly Ser His Met Leu Glu
               20

## Description

### TECHNICAL FIELD OF THE INVENTION

The invention relates to DNA sequences encoding inactive forms of CMH-1, a cysteine protease involved in programmed cell death. The invention further relates to the polypeptides encoded by those DNA sequences. The invention also relates to recombinant DNA molecules comprising these DNA sequences, as well as hosts transformed with such recombinant DNA molecules. The invention further relates to the use of the DNA sequences of this invention in gene therapy for inhibiting apoptosis. The DNA sequences of this invention are also useful in diagnosing cells which have the potential to undergo apoptosis. Finally, the polypeptides encoded by the DNA sequences of this invention are useful in identifying inhibitors of apoptosis.

### BACKGROUND OF THE INVENTION

Programmed cell death (apoptosis) plays an important role in embryonic development, homeostasis, and in diseases such as neurodegenerative disorders, autoimmune diseases and cancer [R. E. Ellis et al., Ann. Rev. Cell Biol., 7, pp. 663-98 (1991)]. Apoptosis is characterized by a set of cellular events including cell shrinkage, chromatin condensation, and DNA fragmentation. Activation of proteases is one of crucial events in the cellular execution of apoptosis (S. J. Martin et al., Cell, 82, pp. 349-52 (1995)].

Genetic studies in the nematode *Caenorhabditis* *elegans* have provided evidence that *Ced*-3 gene is indispensable for cell death during worm development [J. Yuan et al., Cell, 75, pp. 641-52 (1993)]. The CED-3 protein shares 28% sequence identity to mammalian interleukin-1b (IL-1b) converting enzyme (ICE) that cleaves the inactive IL-1b precursor to the proinflammatory cytokine [N. A. Thornberry et al., Nature, 356, pp. 768-74 (1992)]. Both ICE and CED-3 are members of a newly-discovered cysteine protease family that also includes mammalian enzymes Tx/ICH-2/ICEreIII, ICEreIIII, Nedd-2/ICH-1, CPP32/Yama/Apopain, and Mch2 [C. Faucheu et al., EMBO J., 9, pp. 1914-22 (1995); N. A. Munday et al., J. Biol. Chem., 270, pp. 15870-76 (1995); L. Wang et al., Cell, 78, pp. 739-50 (1994); S. Kumar et al., Genes Dev., 8, pp. 1613-26; T. Fernandes-Alnemri et al., J. Biol. Chem., 269, pp. 30761-64 (1994); T. Fernandes-Alnemri et al., Cancer Res., 55, pp. 2737-42 (1995)]. Each of these cysteine proteases is composed of two subunits of approximately 20 kDa and 10 kDa derived from processing of precursor polypeptides. They share conserved amino acid residues for catalysis and binding of P1 aspartate residue (K. P. Wilson et al., Nature, 370, pp. 270-73 (1994); and N. P. C. Walker et al., Cell, 78, pp. 343-52 (1994)].

Overexpression of cysteine proteases in the Ice/Ced-3 gene family leads to apoptosis of transfected fibroblast cells [M. Miura et al., Cell, 75, pp. 653-60 (1993); C. Faucheu et al., EMBO J., 9, pp. 1914-22 (1995); T. Fernandes-Alnemri et al., J. Biol. Chem., 269, pp. 30761-64 (1994); T. Fernandes-Alnemri et al., Cancer Res., 55, pp. 2737-42 (1995); Y. Gu et al., EMBO J., 9, pp. 1923-31 (1995); M. Tewari et al., Cell, 81, pp. 3255-60 (1995)], and ICE inhibitors block cell death of neurons deprived of neurotrophic factors [V. Gaglardini et al., Science, 263, pp. 826-28 (1994); and C. E. Milligan et al., Neuron, 15, pp. 385-93 (1995)]. The involvement of ICE or related proteases has been implicated in Fas- and TNFα-mediated apoptosis (K. Kuida et al., Science, 267, pp. 2000-03 (1995); M. Enari et al., Nature, 375, pp. 78-79 (1995); M. Los et al., Nature, 375, pp. 81-83 (1995); M. Tewari et al., J. Biol. Chem, 270, pp. 3255-3260 (1995); M. Miura et al., Cell, 75, pp. 653-660 (1995)].

Furthermore, ICE deficient mice are resistant to apoptosis induced by an anti-Fas antibody, suggesting a physiological role for ICE in Fas-mediated apoptosis of thymocytes (K. Kuida et al, 1995).

A number of proteins, including poly(ADP-ribose) polymerase (PARP), 70 kDa protein component of the U1 small nuclear ribonucleoprotein, lamin B1, β-actin and topoisomerase I are proteolytically degraded during apoptosis. Most notably, the rapid and complete cleavage of 116-kDa PARP between Asp214 and Gly215 to 31-kDa and 85-kDa polypeptides occurs at the onset of apoptosis [S. H. Kauffman et al., Cancer Res., 53, pp. 3976-85 (1993); Y. A. Lazebnik et al., Nature, 371, pp. 346-47 (1995)].

Some workers have suggested that CPP32 is the cysteine protease that cleaves PARP in a cytosolic extract of apoptotic cells [D. W. Nicholson et al. Nature, 376, pp. 37-43 (1995); Y. A. Lazebnik et al., (1995)]. Cytotoxic T lymphocyte (CTL)-specific granzyme B cleaves and activates CPP32, resulting in PARP cleavage in target cells, suggesting a protease activation cascade involving CPP32 in CTL-mediated apoptosis [A. J. Darmon et al., Nature, 377, pp. 446-48 (1995)]. In addition, CPP32 cleaves and activates sterol responsive element binding proteins (SREBP) that are involved in transcription regulation of genes for low density lipoprotein receptor and 3-hydroxy-3-methylglutaryl CoA Synthase [X. Wang et al., J. Biol. Chem., 270, pp. 18044-50 (1995)].

Finally, human interleukin-1 β converting enzyme like apoptosis proteases -3 and 4 and DNA encoding such polypeptides have been identified (WO 96/13603). These proteases are structurally related to ICE and the DNA sequences and polypeptides described here.

Despite these developments to date, other members of the *Ice/Ced*-3 gene family of cysteine proteases need to be identified and characterized in order to fully understand the mechanisms of apoptosis.

### SUMMARY OF THE INVENTION

The invention provides a complete cDNA sequence encoding a 34-kDa polypeptide that is homologous to CPP32. The full-length cDNA encodes the proenzyme ("activatable") form of a cysteine protease -- CMH-1 -- that is important in programmed cell death ("apoptosis"). The invention also provides cDNA sequences encoding inactive, mutated forms of CMH-1.

The invention also provides recombinant DNA molecules comprising any of the above-described cDNA sequences. The recombinant DNA molecules of this invention are used to transform both prokaryote and eukaryote hosts. Such transformed hosts are also part of this invention. The transformed hosts may be utilized to produce large quantities of the DNA sequences of this invention. Additionally, the transformed hosts of this invention may be utilized to produce any of the polypeptides encoded by those DNA sequences.

The DNA sequences of this invention may be used in both therapeutic and diagnostic applications.

The DNA sequences encoding inactive, mutant forms of CMH-1 are useful in gene therapy to prevent apoptosis by competing with the native, active form of the enzyme in the cell. This therapeutic use is especially useful in preventing neurological diseases.

The DNA sequences of this invention may be used as diagnostic tools to detect and quantify Cmh-1 mRNA levels in various cells. This method may be used to determine the sensitivity of cancer cells to chemotherapeutic treatment. A high Cmh-1 mRNA level suggests that the cell would be responsive to chemotherapy. This method may also be used to detect diseases in cells that do not normally undergo apoptosis, especially cells involved in neurological disease. A high level of Cmh-1 mRNA would be indicative of a disease state.

The Cmh-1 DNA sequences of this invention may also be used to inhibit the expression of CMH-1 polypeptides in a cell through the use of anti-sense technology. Single stranded, antisense DNA can be introduced into cells where it can hybridize to and inhibit the translation of Cmh-1 mRNA. Such methods can be used to prevent undesirable apoptosis, such as is characteristic of neurological diseases.

### BRIEF DESCRIPTION OF THE DRAWINGS

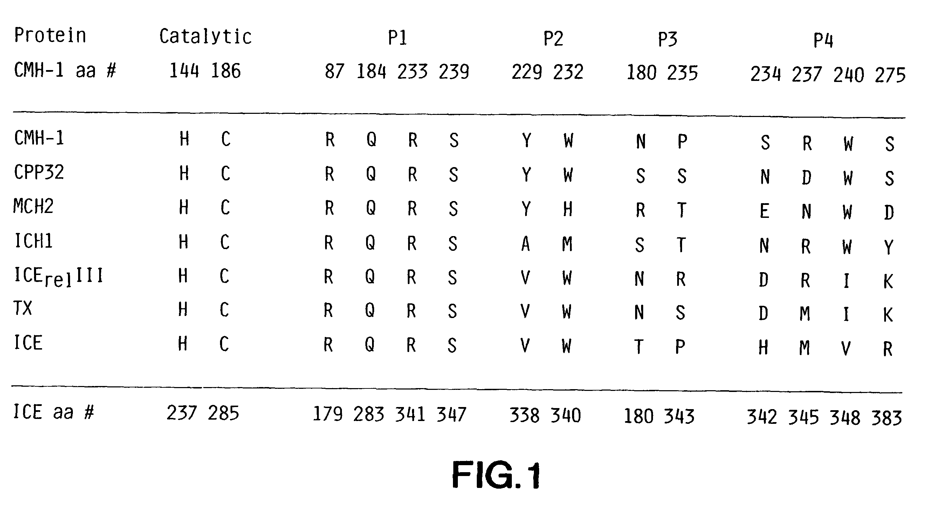

Figure 1 depicts a comparison of the amino acid residues lining each of the substrate side-chain binding pockets of various members of the ICE cysteine protease family.

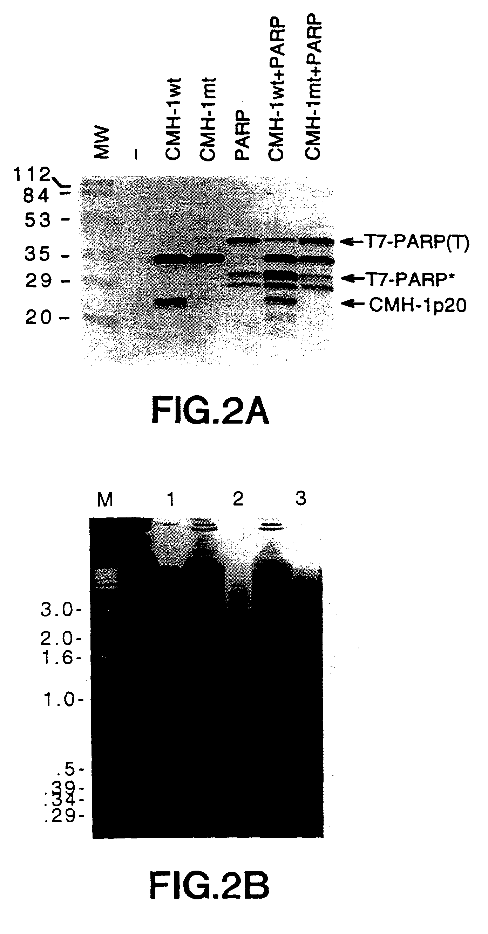

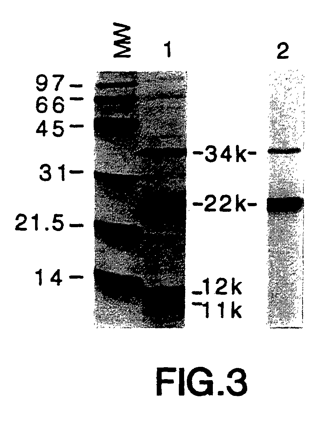

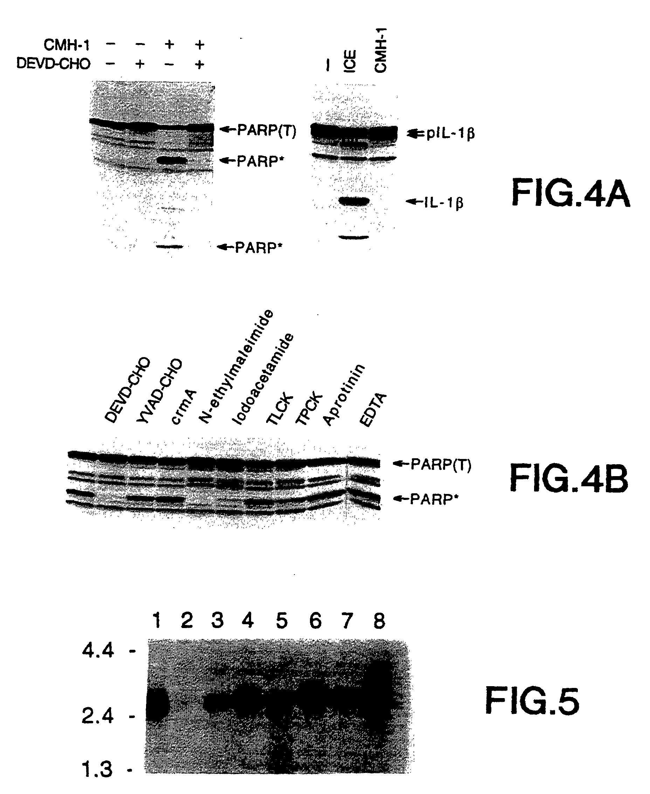

Figure 2, panel A, depicts an immunoblot of extracts of COS cells transfected with plasmids expressing T7-tagged derivatives of an activated form of CMH-1(Ala24-Gln303) (lane "CMH-1wt"), an inactivated mutant of CMH-1(Ala24-Gln303) (lane "CMH-1mt"), a truncated PARP (Gu et al., 1995b) alone (lane "PARP"), truncated PARP and activated CMH-1(Ala24-Gln303) together (lane "CMH-1wt+FARP"), or truncated PARP and the CMH-1 mutant together (lane "CMH-1mt+PARP"). Immunoblotting was carried out using an anti-T7 antibody. Arrows indicate the position of cleaved p20 CMH-1. "PARP(T)" denotes truncated PARP. "PARP(*)" denotes the tagged N-terminal fragment of cleaved PARP.
Figure 2, panel B depicts the induction of apoptosis in transfected COS cells by active or inactive CMH-1, as determined by internucleosomal DNA fragmentation.
Figure 3 depicts the purification of recombinant (His)₆-tagged CMH-1 following Ni-affinity chromatography. Proteins were detected either by Commassie blue staining (lane 1) or by immunoblotting with anti-(His)₆-tag antibody (lane 2).
Figure 4, panel A depicts the ability of recombinant active CMH-1 to cleave PARP in the presence or absence of the tetrapeptide aldehyde inhibitor DEVD-CHO (first four lanes). "PARP(T)" denotes the uncleaved truncated form of PARP. "PARP*" indicates the primary cleavage products (32 and 12 kDa).
Figure 4, panel A, also compares the ability of CMH-1 or ICE to cleave IL-1b precursor (last three lanes). "pIL-1β" denotes the uncleaved precursor. "IL-1β" denotes the mature cleavage product.
Figure 4, panel B depicts the effects of various protease inhibitors on the ability of CMH-1 to cleave ³⁵S-labelled PARP in vitro, as analyzed by SDS-PAGE and fluorography.
Figure 5 depicts a Northern blot analysis of human Cmh-1 in heart (lane 1), brain (lane 2), placenta (lane 3), lung (lane 4), liver (lane 5), skeletal muscle (lane 6), kidney (lane 7), and pancreas (lane 8) using full length CMH-1 cDNA as a probe.

### DETAILED DESCRIPTION OF THE INVENTION

The following definitions are used throughout the specification and the claims.

"Cmh-1" refers to nucleotide sequences, either DNA or RNA, single-stranded or double-stranded that encode a CMH-1 polypeptide. When used to define a double-stranded nucleotide sequence, the term also refers to the anti-sense nucleotide sequence.

"CMH-1" and "CMH-1 polypeptide" refer to a polypeptide which shares at least 80%, and more preferably, at least 90% homology with amino acids 29-303 of SEQ ID NO:2.

An "active CMH-1 polypeptide" is a CMH-1 polypeptide that possesses cysteine protease activity and is capable of cleaving PARP by the assay set forth in Example 4. Examples of active CMH-1 polypeptides of this invention are amino acids 24-303 of SEQ ID NO: 2 and amino acids 29-303 of SEQ ID NO:2.

An "activatable CMH-1 polypeptide" is an inactive form of a CMH-1 polypeptide which, upon cleavage, is converted into an active CMH-1 polypeptide (i.e., a proenzyme form). An example of an activatable CMH-1 polypeptide is SEQ ID NO:2.

An "inactive CMH-1 polypeptide" is a CMH-1 polypeptide that lacks cysteine protease activity, due to a mutation, deletion or substitution. Preferred examples of inactive CMH-1 polypeptides are those which have been mutated at one or more of the following catalytic residues: Cys186, Trp232 or Arg233 present in SEQ ID NO:2.

Additional terms are defined where necessary throughout the application.

According to one embodiment, the invention provides an isolated DNA sequence encoding an active, activatable or inactive CMH-1 polypeptide selected from SEQ ID NO:1; DNA sequences that hybridize to SEQ ID NO:1; and DNA sequences which but for the degeneracy of the genetic code would hybridize to SEQ ID NO:1 or sequences which hybridize thereto and code for a polypeptide having the same amino acid sequences coded for by SEQ ID NO:1 or sequences which hybridize thereto.

As used herein, the term "hybridize to" refers to the ability of a denatured DNA sequence to hydrogen bond to another denatured DNA sequence through complementary base pairs under conditions which allow sequences having at least 80% homology to form such hybrids. Such conditions are well known in the art and are exemplified by salt and temperature conditions substantially equivalent to 5X SSC and 65°C for both hybridization and wash.

Although only a single full-length cDNA sequence is set forth in the attached Sequence Listing, it will be readily apparent to those of skill in the art that any other DNA sequence which encode the same amino acid sequence (SEQ ID NO:2:): are also be part of applicant's invention. Such DNA sequences merely utilize the redundancy of the genetic code to encode the same amino acids. While those specific sequences are not set forth herein due to space considerations, it should be understood that one of ordinary skill in the art could ascertain all of such DNA sequences merely by reference to the genetic code and without the exercise of inventive skill.

In addition to genetically redundant DNA sequences, the invention also includes DNA sequences which encode other amino acid sequences which are at least 80%, and preferably at least 90% homologous to SEQ ID NO:2. Because this aspect of the invention does not require the isolated DNA sequence to encode an active CMH-1, any nucleotides of SEQ ID NO:1 may be modified to produce a DNA sequence of this invention. The identification and isolation of these additional sequences may be achieved by standard DNA library screening techniques (hybridization, PCR) using SEQ ID NO:1 or portions thereof as a probe. Such homologous sequences may be found in any mammalian tissue cDNA library, as well as in insect cDNA libraries, yeast and other fungi cDNA libraries and prokaryotic cDNA libraries.

According to another embodiment, the invention provides an isolated DNA sequence encoding an inactive CMH-1 polypeptide selected from DNA sequences which hybridize to and are at least 80%, homologous to SEQ ID NO:1 and which encode an active site mutation at either amino acid 144 or 186 of SEQ ID NO:2; and DNA sequences which but for the degeneracy of the genetic code would hybridize to and code for a polypeptide having the same amino acid sequence as the former DNA sequences. More preferred are DNA sequences which encode mutations at both amino acid 144 and 186 of SEQ ID NO:1. Even more preferred are DNA sequences which additionally encode mutations at one or more of the following amino acids of SEQ ID NO:2: 87, 184, 233, 239, 229, 232, 180, 235, 234, 237, 240 and 275. Most preferred is a DNA sequence which encodes the following mutations in SEQ ID NO:2: Cys186-Ser, Trp232-Ala and Arg233-Glu.

As set forth above, DNA sequences according to this aspect of the invention may be identified and isolated through standard cDNA library screening. Specific mutations may be introduced into Cmh-1 DNA through the use of site-directed mutagenesis. The translation products of such DNA sequences may then be assayed for (lack of) cysteine protease activity as described below.

According to another embodiment of this invention, any of the DNA sequences described above may be employed in a recombinant DNA molecule. The isolated DNA sequences of this invention may be inserted into any of the numerous commercially or otherwise publicly available cloning vectors. Useful expression vectors, for example, may consist of segments of chromosomal, non-chromosomal and synthetic DNA sequences. Suitable vectors include derivatives of SV40 and known bacterial plasmids, e.g., E.coli plasmids col E1, pCR1, pBR322, pMB9 and their derivatives, plasmids such as RP4; phage DNAs, e.g., the numerous derivatives of phage λ, e.g., NM989, and other phage DNA, e.g., M13 and Filamentous single stranded phage DNA; yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

The choice of cloning vector will, of course, depend upon the cell type(s) to be transformed with the resulting recombinant DNA molecule, as well as the ultimate use of that recombinant DNA molecules (i.e., DNA production, gene therapy, polypeptide expression). Preferred eukaryotic vectors are SV40-derived vectors. Preferred prokaryotic vectors are *E. coli* expression vectors. Preferred viral vectors are modified eukaryotic viral vectors, preferably attenuated adenovirus vectors, that may be used in gene therapy, such as those described in PCT publications WO 94/26915 and WO 94/28938.

Techniques for inserting a DNA sequence of this invention into a vector to produce a recombinant DNA molecule involve standard molecular biological techniques and are well known in the art (see, for example, J. Sambrook, et al., Molecular Cloning, A Laboratory Manual, Cold Spring Harbor Laboratory Press, Cold Spring Harbor, New York (1989)).

According to a preferred embodiment, the recombinant DNA molecule of this invention will additionally comprise an expression control sequence operatively linked to a DNA sequence of this invention. The term "expression control sequence" refers to a DNA sequence that controls and regulates the transcription and translation of another DNA sequence. It includes both 5' and 3' non-coding DNA sequences and optionally includes an ATG start codon.

The term "operatively linked" refers to the positioning of an expression control sequence with respect to a coding DNA sequence of interest such that the expression control sequence controls and regulates the transcription and translation of that DNA sequence. The term "operatively linked" includes having an appropriate start signal (e.g., ATG) in front of the DNA sequence to be expressed and maintaining the correct reading frame to permit expression of the DNA sequence under the control of the expression control sequence and production of the desired product encoded by the DNA sequence. If a gene that one desires to insert into a recombinant DNA molecule does not contain an appropriate start signal, such a start signal can be inserted in front of the gene.

The choice of expression control sequence depends upon the nature of the recombinant DNA molecule, the host that will be transformed by that recombinant DNA molecule, and whether constitutive or inducible expression of a DNA sequence of this invention is desired. Such useful expression control sequences include, for example, the early and late promoters of SV40 or adenovirus, the lac system, the trp system, the TAC or TRC system, the major operator and promoter regions of phage λ, the control regions of fd coat protein, the promoter for 3-phosphoglycerate kinase or other glycolytic enzymes, the promoters of acid phosphatase (e.g., Pho5), the promoters of the yeast α-mating factors, and other sequences known to control the expression of genes of prokaryotic or eukaryotic cells or their viruses, and various combinations thereof.

These recombinant DNA molecules are used to produce CMH-1 polypeptides. Preferred expression control sequences are the SRα promoter, when a eukaryotic vector is employed, and any inducible promoter when a prokaryotic vector is employed. Most preferred are the expression control sequences contained in the commercially available pET-15b *E. coli* vector (Novagen).

The invention also provides host cells transformed by the recombinant DNA molecules of this invention. These hosts may include well known eukaryotic and prokaryotic hosts, such as strains of E.coli, Pseudomonas, Bacillus, Streptomyces, fungi such as yeasts, and animal cells, such as CHO, R1.1, B-W and L-M cells, African Green Monkey kidney cells (e.g., COS 1, COS 7, BSC1, BSC40, and BMT10), insect cells (e.g., Sf9), and human cells and plant cells in tissue culture. Eukaryotic cells may harbor the recombinant DNA molecules of this invention as an extrachromosomal element or incorporate all or part of it into the host chromosome.

It will be understood that not all vectors, expression control sequences and hosts will function equally well to replicate and/or express the DNA sequences of this invention. Neither will all hosts function equally well with the same expression system. However, one skilled in the art will be able to select the proper vectors, expression control sequences, and hosts without undue experimentation to accomplish the desired expression without departing from the scope of this invention. For example, in selecting a vector, the host must be considered because the vector must function in it. The vector's copy number, the ability to control that copy number, and the expression of any other proteins encoded by the vector, such as antibiotic and other selective markers, will also be considered.

In selecting an expression control sequence, a variety of factors will normally be considered. These include, for example, the relative strength of the system, its controllability, and its compatibility with the particular DNA sequence or gene to be expressed, particularly as regards potential secondary structures. Suitable unicellular hosts will be selected by consideration of, e.g., their compatibility with the chosen vector, their ability to fold proteins correctly, and their fermentation requirements, as well as the toxicity to the host of the product encoded by the DNA sequences to be expressed, and the ease of purification of the expression products.

Considering these and other factors a person skilled in the art will be able to construct a variety of vector/expression control sequence/host combinations that will replicate and/or express the DNA sequences of this invention on fermentation or in large scale animal culture.

Methods for transforming cells with recombinant DNA molecules are well known in the art (see, for example, J. Sambrook, et al., Molecular Cloning, A Laboratory Manual, Cold Spring Harbor Laboratory Press, Cold Spring Harbor, New York (1989)). Any of those methods may be employed to produce the transformed hosts of this invention. Identification of transformed hosts may be achieved by assaying for the presence of Cmh-1 DNA, Cmh-1 RNA or a CMH-1 polypeptide. Additionally, transformants may be identified by growth in selective media. For this assay, the gene necessary for growth in selective media is cotransfected into the cell either on the same or a different recombinant DNA molecule as the Cmh-1 DNA and is expressible in that cell. It will, of course, be obvious that the gene for selective growth should not be present in the untransformed cell.

The transformed hosts of this invention may be employed to produce either large quantities of Cmh-1 DNA sequences of this invention and/or a CMH-1 polypeptide encoded thereby. In order to produce large quantities of Cmh-1 DNA, the host, preferably a prokaryotic host, is grown in a medium and under conditions that promote DNA replication and cell division. Any complete media routinely used to grow bacteria is suitable for this purpose. Following growth, the transformed cells are separated from the growth medium and plasmid DNA is then isolated from the cells by standard and well-known techniques. Cmh-1 DNA may then be excised from the plasmid through the use of restriction endonucleases.

In this embodiment, the preferred host is a mammalian cell. The transformed host should be grown in a medium that promotes expression of the CMH-1 polypeptide-encoding DNA sequence present in that host. If expression of that DNA sequence is under the control of a constitutive promoter, any standard growth medium is suitable. If the Cmh-1 DNA is under the control of an inducible promoter, the growth medium should be supplemented with a compound that induces expression or growth conditions should be altered so as to induce expression (i.e., change in growth temperature). Following expression, the transformed cells are separated from the growth medium, lysed and the CMH-1 polypeptide purified by standard methods.

In order to determine the presence of an active CMH-1 polypeptide, a protease activity assay is used. Such an assay measures the ability of the polypeptide to cleave natural of synthetic substrates. Active CMH-1 polypeptides cleave Asp-X bonds. The preferred natural substrate for these assays is poly(ADP-ribose) polymerase ("PARP"). Synthetic substrates useful in these assays are peptides containing an Asp-X bond. A preferred assay for detecting active CMH-1 polypeptide is described in Example 4.

In addition to the CMH-1 polypeptides, the invention also provides antibodies to CMH-1 polypeptides. The generation of antibodies may be achieved by standard methods in the art for producing both polyclonal and monoclonal antibodies using a CMH-1 polypeptide or fragment thereof as antigen. Such antibodies or active fragments thereof (such as Fab fragments), may be used to assay *in vivo* or *in vitro* levels of CMH-1 polypeptide. Increased *in vivo* levels of CMH-1 polypeptides may be indicative of the onset of apoptosis.

Antibodies to CMH-1 polypeptides also have potential therapeutic use in preventing apoptosis in a patient. Alternatively, the CMH-1 antibodies may be used to regulate cholesterol levels in a patient. As stated previously, the cysteine proteases involved in apoptosis also activate sterol responsive element binding proteins, which in turn regulate transcription of genes encoding the LDL receptor and 3-hydroxy-3-methylglutaryl CoA Synthase (X. Wang et al. (1995)).

Such antibodies may be directed to their target cells through conjugation to cell-specific molecules. The conjugation of such antibodies and the identity of cell-specific targeting molecules is known in the art.

Finally, CMH-1 polypeptide antibodies may be employed to purify CMH-1 polypeptides from either native sources or transformed hosts expressing CMH-1 polypeptides. For example, the antibodies can be conjugated by standard techniques to an insoluble matrix to form an immunoaffinity resin.

The Cmh-1 DNA sequences of this invention, or fragments thereof, may be used to identify homologous DNA sequences so as to potentially identify other members of this cysteine protease family. The DNA sequences of this invention are useful in a number of therapeutic and diagnostic applications.

Gene delivery systems other than viruses can also be employed in the methods of this invention. For example, the gene to be transferred may be packaged in a liposome. When cells are incubated with DNA-encapsidated liposomes, they take up the DNA and express it. To form these liposomes, one mixes the DNA of an expression vector which expresses the gene to be transferred with lipid, such as N-[1-(2,3,dioleyloxy)propyl]-N,N,N-trimethylammonium chloride (DOTMA) in a suitable buffer, such as Hepes buffered saline. This causes the spontaneous formation of lipid-DNA complexes (liposomes) which can be employed in the methods of this invention [P.L. Felgner et al., Proc. Natl. Acad. Sci. USA, 84, pp. 7413-17 (1987)].

Another gene delivery system that may be utilized in this invention is DNA-protein complexes. The formation of these complexes is described in United States patent 5,166,320. Specifically, these complexes comprise the gene to be transferred (together with promoter, enhancer sequences and other DNA necessary for expression in the target cell) linked via a suitable polymer, such as polylysine, to a polypeptide ligand for a receptor or other cell surface protein. This complex is taken up by the target cells via endocytosis after the ligand binds to the cell surface receptor. The DNA is then cleaved from the rest of the complex via intracellular enzymes which cut the polymer linker. Such complexes are particularly useful in targeting specific cell types. For example, cancer cells may be specifically targeted through the use of proteins which specifically recognize tumor cell surface markers or receptors.

The preferred use according to this embodiment is in the treatment of cancer in a patient. The term "patient," as used herein refers to any mammal, especially humans. Cancer cells transformed with a DNA sequence encoding an active or activatable CMH-1 polypeptide will express that DNA, which in turn, will cause cell death.

DNA sequences encoding inactive CMH-1 polypeptides are useful in gene therapy to inhibit apoptosis. According to this embodiment, the inactive CMH-1 polypeptide encoded by this DNA would competitively compete for substrate with native, active CMH-1. This would decrease the CMH-1 activity in the cell, inhibiting apoptosis. Preferred cells to be treated by this method are nerve cells.

The DNA sequences of this invention may also be used as anti-sense inhibitors of CMH-1 polypeptide mRNA and thus inhibitors of apoptosis. In this embodiment, single stranded, anti-sense Cmh-1 DNA is introduced into cells by standard techniques. Once inside the cell, the DNA binds to Cmh-1 mRNA and prevents it from being translated.

According to an alternate embodiment, the DNA sequences of this invention may be used as a diagnostic tool to determine Cmh-1 mRNA levels in cells. This may be achieved by standard Northern blot or dot blotting techniques that are well known in the art. For example, a low level of Cmh-1 mRNA in cancer cells is indicative that those cells will respond to chemotherapy. In normal cells, high levels of Cmh-1 mRNA may be predictive of disease, especially in nerve and other neurological cells.

In order that the invention described herein may be more fully understood, the following examples are set forth. It should be understood that these examples are for illustrative purposes only and are not to be construed as limiting this invention in any manner.

### EXAMPLE 1

### Cloning of CMH-1 cDNA

We initially screened the GenBank EST database maintained by the National Center for Biotechnology Information (NCBI) for cDNA clones encoding ICE-like polypeptides using the Blast algorithm [S. F. Altshul et al., J. Mol. Biol., pp. 403-10 (1990)]. We generated initial multiple sequence alignments of known ICE family protein peptide sequences using the MACAW program (G. D. Schuler et al., Proteins: Structure, Function, and Genetics, 9, pp. 180-90 (1991)] and later refined by the combination of the PILEUP algorithm (Genetics Computer Group, 1995) and pairwise comparison.

Through this screen, we identified a partial human cDNA clone deposited by the Washington University-Merck EST project (accession number T50828) which had a partial open reading frame with limited similarity to our consensus ICE sequence and a 3' untranslated region. We obtained the partial human cDNA clone 72778 corresponding to this EST (expressed sequence tag) sequence through the IMAGE Consortium (Lawrence Livermore National Laboratory).

In order to generate a full-length cDNA clone, we ligated a spleen cDNA pool with a DNA adaptor (Clontech, Palo Alto, CA) and subsequently performed PCR using a primer to the 5' adaptor (SEQ ID NO:3: 5'-CCATCCTAATACGACTCACTATAGGGC)and a primer to the EST T50828 sequence (SEQ ID NO:4: 5'-GCAAACTCTGTCAATTCACCC). Through this technique, we were able to generate a 0.8 kb SacI-HgiAI PCR fragment containing the 5' coding region. We then ligated this fragment to the 1.55 kb HgiAI-KpnI fragment from the 72778 clone containing the 3' region subcloned the resulting DNA into pBluescript SK (Stratagene, La Jolla CA) at SacI/KpnI to generate the full length cDNA.

We sequenced the resulting cDNA, named Cmh-1 (CPP32/Mch2 homolog-1), on both strands by ABI Prism Dye Deoxy Terminator sequencing using the ABI 373 DNA Sequencer. The cmh-1 sequence is set forth in the Sequence Listing as SEQ ID NO:1.

Analysis of the full length cDNA sequence identified a single open reading frame of 303 amino acids encoding a protein of 34 kDa, which we have named CMH-1 The encoded protein is set forth in the Sequence Listing as SEQ ID NO:2. We identified the catalytic residues as His144 and Cys186 in SEQ ID NO:2.

CMH-1 was found to have 52% identity to CPP32 identified by T. Fernandes-Alnemri et al. (1994). In addition, there was sequence similarity with all members of the ICE/Ced3 gene family. Overall, the polypeptide can be divided into three domains, roughly corresponding to the P15, P20, and P10 subdomains of ICE. In CMH-1 these domains are 2, 21, and 11 kD, respectively.

There is strong similarity between CMH-1 and the sequence that comprises the core of the ICE structure, as well as a number of residues surrounding the active site Cys285 and His237 in the ICE crystal structure [K. P. Wilson et al., Nature, (1994); N. P. C. Walker et al., Cell, (1994)]. Superposition of the CMH-1 sequence onto this ICE structure allowed the identification of a number of other residues located around the protease active site (Fig. 1).

Interestingly, all of the residues which line the substrate binding pocket at the P1 position are identical with those found in ICE, including Arg87 and Arg233, corresponding to ICE Arg179 and Arg341. This strongly suggests that CMH-1 has substrate specificity for Asp in this position, as seen in other members of this family.

There is much less sequence conservation in the binding pockets corresponding to the P3-P4 residues between CMH-1 and its closest homolog CPP32, suggesting that CMH-1 has a different substrate(s).

### EXAMPLE 2

### Expression And Processing of cmh-1 in COS cells

We used transient expression of CMH-1 in COS cells to determine if CMH-1 has the predicted cysteine protease activity. Removal of the pro-domain from the CPP32 precursor occurs between Asp28 and Ser29 [D. W. Nicholson et al., Nature, (1995)]. For CMH-1, we estimated the position at which the prodomain removal might occur as Asp23-Ala24.

We therefore produced a cDNA fragment encoding an N-terminally T7-tagged CMH-1 lacking the first 23 amino acid residues by PCR using the following primers: SEQ ID NO:5: 5'-GCTGCAGTCTAGAGCTCCATGGCTAGCATGACTGGTG GACAGCAAATGGGTGCTAAGCCAGACCGGTCCTCG-3' and SEQ ID NO:6: 5'-GAATTCTAGATTCTATTGACTGAAGTAGAGTTC-3'. We digested the resulting amplified DNA with XbaI and subcloned it into an XbaI-cut SV40-based expression vector under the control of SRα promoter (pcDLSRα) [Y. Takebe et al., Mol. Cell. Biol., 8, pp. 466-72 (1988)].

We produced a corresponding inactive mutant CMH-1 containing a Cys186→Ser, a Trp232→Ala and a Arg233→Glu mutation (C186S/W232A/R233E) by site-directed mutagenesis using oligonucleotides SEQ ID NO:7: 5'-CATTCAGGCTAGCCGAGGGAC and SEQ ID NO:8: 5'-GCTATTACTCAGCTGAGAGCCCAGGA and following well-known techniques [T. A. Kunkel, Proc. Natl. Acad. Sci. USA, 82, pp. 488-92 (1985); Y. Gu et al., EMBO J., (1995), the disclosures of which are herein incorporated by reference]. These substitutions were at the conserved residues for catalysis and P1 aspartate binding.

We transiently transfected COS cells using the DEAE-dextran method as described by Y. Gu et al., EMBO J., (1995). COS cells were grown in 6 well culture plates and transfected with 3µg of plasmid DNA containing wither the active or inactive CMH-1 coding sequences. We harvested the cells 24 hours after transfection.

Transfection of the expression plasmid in COS cells results in high expression and processing of the truncated CMH-1 protein. Immunoblotting of the COS cell lysates with an anti-T7 antibody detected the unprocessed precursor with an apparent molecular weight of 34 kDa and a processed product with an apparent molecular weight of 22 kDa (Fig. 2, panel A).

When we co-expressed T7-CMH-1 with a 43 kDa truncated form of T7-tagged PARP, the PARP was cleaved into a T7-tagged 31-kDa fragment, similar to the cleavage of PARP by CPP32, ICE, Tx, and Nedd-2 [Y. Gu et al., J. Biol. Chem., 270, pp. 18715-18 (1995)]. The inactive mutant (C186S/W232A/R233E) was defective in both autoprocessing and PARP cleavage.

Similar to other ICE-like cysteine proteases, we found that overexpression of the CMH-1 protein induces cell apoptosis, as demonstrated by the presence of cell shrinkage and DNA fragmentation in the transfected cells (Fig. 2, panel B). It was apparent that induction of apoptosis was dependent on the presence of cysteine protease activity because expression of the active site mutant did not result in apoptosis of transfected COS cells (Fig. 2, panel B).

### EXAMPLE 3

### Expression and Purification of Recombinant CMH-1 In E. coli

To further characterize the activity of CMH-1 protein, we expressed an N-terminal (His)6-tagged CMH-1(Ala24-Gln303) in *E. coli*. We constructed an expression plasmid for the N-terminal (His)₆-tagged CMH-1(Ala24-Gln303) by introducing in-frame XhoI sites to 5' and 3' ends of Cmh-1 cDNA using PCR with the following primers:
SEQ ID NO:9: 5' CGGCTCGAGGCTAAGCCAGACCGGTCCTCG and SEQ ID NO:10: 5' GGGCTCGAGCTATTGACTGAAGTAGAGTTC. The resulting XhoI fragment was ligated into an XhoI-cut pET-15b inducible E. coli expression vector (Novagen, Madison, WI). The resulting plasmid encodes a polypeptide of 303 amino acids with the 23-residue peptide SEQ ID NO:11: (MGSSHHHHHHSSGLVPRGSHMLE, where LVPRGS (amino acids 14-19 of SEQ ID NO:11) represents a thrombin cleavage site fused in frame to the N-terminus of CMH-1 starting at Ala24, as confirmed by DNA sequencing as well as by N-terminal sequencing of the expressed proteins.

*E. coli* strain BL21(DE3) carrying this plasmid was induced with 0.8 mM IPTG, harvested and lysed in a microfluidizor in a buffer containing 20 mM sodium phosphate (pH 6.8), 300 mM NaCl, 2 mM DTT, 10% glycerol, 0.4 mM PMSF, 2.5 µg/ml leupeptin. We cleared the lysates by centrifugation at 100,000 x g for 30 minutes.

The supernatant containing soluble CMH-1 were loaded onto a 0.5-ml nickel-NTA column (Qiagen) and washed with the same buffer. The column was then eluted with increasing concentrations of imidazole in the same buffer, and CMH-1 protein was eluted at 100-200 mM to give a yield of approximately 5 mg of protein per liter of culture. Imidazole was then removed by dialysis.

The purified protein fraction contained three major polypeptides of approximately 34 kDa, 22 kDa, and 12 kDa (Figure 3). An 11 kDa polypeptide was also present in some preparations.

Immunoblotting of the *E. coli* expressed (His)₆-tagged CMH-1 protein with an anti-(His)6-tag antibody were carried out as described by Gu et al., J. Biol. Chem. (1995). The N-terminus of each of these peptides was analyzed using the ABI 477A Protein Sequencer (Applied Biosystems, Inc., Foster City, CA).

These studies indicated that the 34 kDa and 22 kDa polypeptides contain the (His)6-tag and the predicated N-terminal sequence of the truncated CMH-1 starting at Ala24. The N-terminus of 12 kDa and 11 kDa polypeptides start at Ser199 and Ala207, respectively, both of which are preceded by an Asp residue, suggesting that autoprocessing of the CMH-1(Ala24-Gln303) protein occurs predominantly at the Asp198-Ser199 site and to a lesser extent at the Asp206-Ala207 site. The 22 kDa and 12 kDa proteins are the autoprocessing products of the full length 34 kDa protein. They represent the two subunits of the CMH-1 protease.

### EXAMPLE 4

### In vitro cleavage of PARP by CMH-1

In order to determine the activity of purified CMH-1, we determined whether the protein could cleave ³⁵[S]-labeled pro-IL-1b and PARP and compared its cleavage activity to purified ICE. We prepared ³⁵[S]-labeled PARP and IL-1b precursor (pro-IL-1b) by *in vitro* transcription and translation. Cleavage experiments were carried out as described previously [Y. Gu et al., J. Biol. Chem. (1995)]. Cleavage products were analyzed by SDS-PAGE and fluorography as described in that reference.

The results demonstrated that CMH-1 cleaved PARP but not pro-IL-1b. Less than 3 nM CMH-1 was required to completely process PARP within 30 minutes (Fig. 4, panel A). This efficiency was significantly higher than that of ICE in cleaving the same substrate under identical conditions, and was similar to the activity of CPP32 against PARP (results not shown) or ICE against pro-IL-lb [Y. Gu et al., J. Biol. Chem., (1995)].

The cleavage activity of CMH-1 was inhibited by the tetrapeptide inhibitor DEVD-CHO [D. W. Nicholson et al., Nature, (1995); X. Wang et al., J. Biol. Chem. (1995)], as well as by the cysteine-alkylating reagents N-ethylmaleimide and iodoacetamide, but not by the ICE inhibitors YVAD-CHO [N. A. Thornberry et al., Nature, (1993); Y. Gu et al., J. Biol. Chem. (1995)] or crmA (a viral protein that is a specific ICE inhibitor) or inhibitors of serine proteases or metalloproteases (Fig. 4, panel B). These results demonstrate that CMH-1 is a cysteine protease with properties closer to CPP32 than to ICE. This was consistent with the relative sequence identity between CPP32 and cmh-1.

### EXAMPLE 5

### Northern blot analysis of CMH-1 mRNA

We investigated the expression of Cmh-1 mRNA in adult HUMAN tissues by Northern blotting using a Cmh-1 specific cDNA probe. A human multiple tissue RNA blot (MTN blot, Clontech) containing 2µg/lane of poly (A)⁺RNA was hybridized to the ³²[P]-labeled probe of full length Cmh-1 cDNA in 50% formamide, 10x Denhardts, 2% SDS, 5x SSPE and 100 µg/ml sheared and denatured ssDNA at 42°C for 16hrs. The blot was washed in 2x SSC, 0.1% SDS at room temperature for 40 minutes and at 0.1x SSC, 0.1% SDS at 42°C for 20 minutes and exposed to Kodak XAR-5 film for 18 hours.

A 2.5 kb mRNA was detected in tissues including pancreas, lung, placenta, kidney, muscle and heart. Notably, the expression of Cmh-1 was low in liver and almost undetectable in brain (Figure 5). This ubiquitous expression of Cmh-1 in a variety of tissues is similar to that of CPP32 (data not shown).

## Claims

1. An isolated DNA sequence selected from the group consisting of a DNA sequence which hybridizes to and which shares at least 80% homology with SEQ ID NO:1, wherein, upon expression, said DNA sequence encodes an inactive CMH-1 polypeptide, wherein the polypeptide contains a mutation in at least one of the positions 144 or 186 of SEQ. ID NO:2.

2. The DNA sequence according to claim 1, which upon expression, encodes an inactive CMH-1 polypeptide, wherein the inactive CMH-1 polypeptide has a mutation in at least one amino acid selected from the group comprising;
a) 87;
b) 184;
c) 233;
d) 239;
e) 229;
f) 232;
g) 180;
h) 235;
i) 234;
j) 237;
k) 240; and
l) 275 of SEQ.ID NO:2.

3. The DNA sequence according to claim 1 which upon expression, encodes an inactivate CMH-1 polypeptide, wherein the inactive CMH-1 polypeptide has the amino acid Serine at position 186, Alanine at position 232 and Glutamine at position 233 of SEQ.ID NO:2.

4. A recombinant DNA molecule comprising a DNA sequence according to any one of claims 1-3.

5. The recombinant DNA molecule according to claim 4, wherein said DNA sequence is operatively linked to an expression control sequence, said DNA sequence capable of being expressed in a host cell transformed with said recombinant DNA molecule.

6. A prokaryotic or eukaryotic host cell transformed with a recombinant DNA molecule according to claim 4.

7. A prokaryotic or eukaryotic host cell transformed with a recombinant DNA molecule according to claim 5.

8. The host cell according to claim 6 or 7, wherein said host cell is a mammalian cell.

9. The host cell according to claim 6 or 7, wherein said host cell is a E. coli.

10. A method of producing a DNA sequence encoding a CMH-1 polypeptide according to any one of claims 1-3 comprising the steps of:
(a) incubating a host cell according to claim 6 or 7 in a growth medium;
(b) separating said cells from said medium; and
(c) isolating said DNA sequence from said cells.

11. A method for producing a CMH-1 polypeptide comprising the steps of:
(a) incubating a host cell according to claim 7 in a growth medium under conditions which promote expression of said CMH-1 polypeptide;
(b) separating said cells from said medium; and
(c) isolating said polypeptide from said cells.

12. A CMH-1 polypeptide encoded by a DNA sequence according to any one of claims 1-3.

13. The use of DNA according to claim 1 for the manufacture of a medicament for decreasing CMH-1 activity in a patient.

## Patentansprüche

1. Isolierte DNS-Sequenz ausgewählt aus der Gruppe bestehend aus einer DNS-Sequenz die mit SEQ ID NO:1 hybridisiert und eine Homologie von mindestens 80% zu dieser aufweist, **dadurch gekennzeichnet, dass** diese DNS-Sequenz bei Expression für ein inaktives CMH-1 Polypeptid kodiert, wobei das Polypeptid mindestens in einer der Positionen 144 oder 186 der SEQ ID NO: 2 eine Mutation aufweist.

2. DNS-Sequenz gemäß Anspruch 1, welche bei Expression für ein inaktives CMH-1 Polypeptid kodiert, **dadurch gekennzeichnet, dass** das inaktive CMH-1 Polypeptid eine Mutation in mindestens einer Aminosäure ausgewählt aus der Gruppe umfassend;
a) 87;
b) 184;
c) 233;
d) 239;
e) 229;
f) 232;
g) 180;
h) 235,
i) 234;
j) 237;
k) 240 und
l) 275 der SEQ ID NO:2 aufweist.

3. DNS-Sequenz gemäß Anspruch 1, welche bei Expression für ein inaktives CMH-1 Polypeptid kodiert, **dadurch gekennzeichnet, dass** das inaktive CMH-1 Polypeptid in der Position 186 der SEQ ID NO:2 die Aminosäure Serin, in Position 232 die Aminosäure Alanin und in Position 233 die Aminosäure Glutamin aufweist.

4. Rekombinantes DNS-Molekül, das eine DNS-Sequenz gemäß einem der Ansprüche 1 bis 3 umfasst.

5. Rekombinantes DNS-Molekül gemäß Anspruch 4, **dadurch gekennzeichnet, dass** diese DNS-Sequenz operativ mit einer Expressions-Kontrollsequenz verbunden ist, und die besagte DNS-Sequenz von einer Wirtszelle, die mit der besagten DNS-Sequenz transformiert wurde, exprimiert werden kann..

6. Prokaryotische oder eukaryotische Wirtszelle, die mit einem rekombinanten DNS-Molekül gemäß Anspruch 4 transformiert wurde.

7. Prokaryotische oder eukaryotische Wirtszelle, die mit einem rekombinanten DNS-Molekül gemäß Anspruch 5 transformiert wurde.

8. Wirtszelle gemäß Anspruch 6 oder 7, **dadurch gekennzeichnet, dass** diese Wirtszelle eine Säugetierzelle ist.

9. Wirtszelle gemäß Anspruch 6 oder 7, **dadurch gekennzeichnet, dass** die Wirtszelle E. coli ist.

10. Verfahren zur Herstellung einer für ein CMH-1-Polypeptid kodierenden DNS-Sequenz gemäß einem der Ansprüche 1 bis 3, Schritte umfassend, bei denen man:
(a) eine Wirtszelle gemäß Anspruch 6 oder 7 in einem Kulturmedium inkubiert;
(b) die Zellen vom Kulturmedium abtrennt; und
(c) die DNS-Sequenz aus den Zellen isoliert.

11. Verfahren zur Herstellung eines CMH-1 Polypeptids, Schritte umfassend, bei denen man:
(a) eine Wirtszelle gemäß Anspruch 7 in Kulturmedium unter Bedingungen, welche die Expression des besagten CMH-1 Polypeptids fördern, inkubiert;
(b) die Zellen vom Kulturmedium abtrennt; und
(c) das Polypeptid aus den Zellen isoliert.

12. CMH-1 Polypeptid, welches von einer DNS-Sequenz gemäß einem der Ansprüche 1 bis 3 kodiert wird.

13. Verwendung von DNS gemäß Anspruch 1 zur Herstellung eines Medikamentes zur Reduktion der CMH-1-Aktivität in einem Patienten.

## Revendications

1. Séquence isolée D'ADN sélectionnée dans le groupe consistant en une séquence d'ADN qui s'hybride à et qui partage au moins 80% d'homologie avec SEQ ID NO :1 où, lors de l'expression, ladite séquence d'ADN code pour un polypeptide CMH-1 inactif, où le polypeptide contient une mutation à au moins l'une des positions 144 ou 186 de SEQ. ID NO : 2.

2. Séquence d'ADN selon la revendication 1, qui lors de l'expression, code pour un polypeptide CMH-1 inactif où le polypeptide CMH-1 inactif a une mutation dans au moins un acide aminé sélectionné dans le groupe comprenant :
a) 87 ;
b) 184 ;
c) 233 ;
d) 239 ;
e) 229 ;
f) 232 ;
g) 180 ;
h) 235 ;
i) 234 ;
j) 237 ;
k) 240 ; et
l 275 de SEQ.ID NO : 2.

3. Séquence d'ADN selon la revendication 1 qui, lors de l'expression, code pour un polypeptide CMH-1 inactivé où le polypeptide CMH-1 inactif a l'acide aminé Serine à la position 186, Alanine à la position 232 et Glutamine à la position 233 de SEQ.ID NO : 2.

4. Molécule d'ADN recombinant comprenant une séquence d'ADN selon l'une quelconque des revendications 1-3.

5. Molécule d'ADN recombinant selon la revendication 4, où ladite séquence d'ADN est opérativement enchaînée à une séquence de contrôle d'expression, ladite séquence d'ADN capable d'être exprimée dans une cellule hôte transformée avec ladite molécule d'ADN recombinant.

6. Cellule hôte procaryote ou eucariote transformée avec une molécule d'ADN recombinant selon la revendication 4.

7. Cellule hôte procaryote ou eucariote transformée par une molécule d'ADN recombinant selon la revendication 5.

8. Cellule hôte selon la revendication 6 ou 7, où ladite cellule hôte est une cellule mammalienne.

9. Cellule hôte selon la revendication 6 ou 7, où ladite cellule hôte est E. coli.

10. Méthode de production d'une séquence d'ADN codant pour un polypeptide CMH-1 selon l'une quelconque des revendications 1-3, comprenant les étapes de :
(a) soumettre à incubation une cellule hôte selon la revendication 6 ou 7 dans un milieu de croissance ;
(b) séparer lesdites cellules dudit milieu ; et
(c) isoler ladite séquence d'ADN desdites cellules.

11. Méthode de production d'un polypeptide CMH-1 comprenant les étapes de :
(a) soumettre à incubation une cellule hôte selon la revendication 7 dans un milieu de croissance dans des conditions qui favorisent l'expression dudit polypeptide CMH-1 ;
(b) séparer lesdites cellules dudit milieu ; et
(c) isoler ledit polypeptide desdites cellules.

12. Polypeptide CMH-1 codé par une séquence d'ADN selon l'une quelconque des revendications 1-3.

13. Utilisation d'un ADN selon la revendication 1 pour la fabrication d'un médicament pour diminuer l'activité de CMH-1 chez un patient.
